# EUROPEAN PATENT SPECIFICATION

(11) **EP 3 507 381 B1**
(45) Date of publication and mention of the grant of the patent: **21.10.2020**
(21) Application number: 17764365.7
(22) Date of filing: 04.09.2017
(51) Int. Cl.: C12Q 1/6869, C12Q 1/6818, C12Q 1/6823

(54) **SINGLE NUCLEOTIDE DETECTION METHOD AND ASSOCIATED PROBES**
EINZELNUKLEOTIDDETEKTIONSVERFAHREN UND ZUGEHÖRIGE SONDEN
PROCÉDÉ DE DÉTECTION D'UN NUCLÉOTIDE SIMPLE ET SONDES ASSOCIÉES

(30) Priority: 02.09.2016 EP 16187112; 09.11.2016 GB 201618915
(43) Date of publication of application: 10.07.2019
(73) Proprietor: Base4 Innovation Limited, Cambridge, Cambridgeshire CB3 0FA (GB)
(72) Inventor: BALMFORTH, Barnaby, Cambridge Cambridgeshire CB3 0FA (GB); FRAYLING, Cameron Alexander, Cambridge Cambridgeshire CB3 0FA (GB)
(74) Representative: Lau, Sarah Jane
(86) International application number: PCT/EP2017/072063
(87) International publication number: WO 2018/042028

(56) References cited:
- WO-A1-2014/053853
- WO-A1-2016/012789
- WO-A2-2006/088911
- US-A- 5 919 630
- CUI WANLING ET AL: "A dual amplification fluorescent strategy for sensitive detection of DNA methyltransferase activity based on strand displacement amplification and DNAzyme amplification", BIOSENSORS AND BIOELECTRONICS, vol. 77, 22 October 2015 (2015-10-22), pages 650-655, XP029311813, ISSN: 0956-5663, DOI: 10.1016/J.BIOS.2015.10.040

## Description

This invention relates to a method and associated biological probes for detecting and characterising single nucleotides. It is especially suitable for use in the sequencing of DNA or RNA.

Next generation sequencing of genetic material is already making a significant impact on the biological sciences in general and medicine in particular as the unit cost of sequencing falls in line with the coming to market of faster and faster sequencing machines.

In our previous applications WO 2014/053853, WO 2014/053854, WO2014/167323, WO2014/167324 and WO2014/111723 we have described a new sequencing method which involves progressive digestion of a polynucleotide analyte to generate an ordered stream of single nucleotides, preferably a stream of single nucleoside triphosphates, each of which can be captured one-by-one into corresponding droplets in a microdroplet stream. Thereafter, each droplet can be chemically and/or enzymatically manipulated to reveal the particular single nucleotide it originally contained. In one embodiment, these chemical and/or enzymatic manipulations comprise a method involving the use of one or more two-component oligonucleotide probe types each of which is adapted to be able to selectively capture one of the single nucleotide types from which the analyte is constituted. Typically, in each of such probe types, one of the two oligonucleotide components comprises characteristic fluorophores and in the probe's unused state the ability of these fluorophores to fluoresce remains extinguished by virtue of the presence of quenchers located close-by or by self-quenching. In use, when the probe has captured its corresponding single nucleotide, it is rendered susceptible to subsequent exonucleolysis thereby liberating the fluorophores from the quenchers and/or each other enabling them to fluoresce freely. By this means, the original single nucleotide present in each droplet can be inferred indirectly by spectroscopic means.

Variants of this method have been described in other of our pending applications including WO201405385 and WO2016012789; the latter involving the use of a three-component probe system. In particular, WO2016012789 describes an improved method characterised by the steps of (1) generating a stream of single nucleoside triphosphates by progressive digestion of the nucleic acid; (2) producing at least one substantially double-stranded oligonucleotide used probe by reacting in the presence of a polymerase and a ligase at least one of the single nucleoside triphosphates with a corresponding probe system comprising (a) a first single-stranded oligonucleotide labelled with e.g. characteristic fluorophores in an undetectable state and (b) second and third single-stranded oligonucleotides capable of hybridising to complementary regions on the first oligonucleotide; (3) digesting the used probe with an enzyme having double-stranded exonucleolytic activity to yield the fluorophores in a detectable state and a single-stranded fourth oligonucleotide which is at least in part the sequence complement of the first oligonucleotide; (4) reacting the fourth oligonucleotide with another first oligonucleotide to produce a substantially double-stranded oligonucleotide product corresponding to the used probe; (5) repeating steps (3) and (4) in a cycle and (6) detecting the fluorophores released in each iteration of step (3). This method has the advantage that by iterating steps (3) and (4) in a cycle the fluorescence signal can be made to grow strongly thereby improving the overall sensitivity and therefore reliability of nucleotide detection. In one embodiment, the second and third oligonucleotides are linked so that, after nucleotide capture, they form a closed-loop single-stranded oligonucleotide component which is advantageously resistant to exonucleolysis.

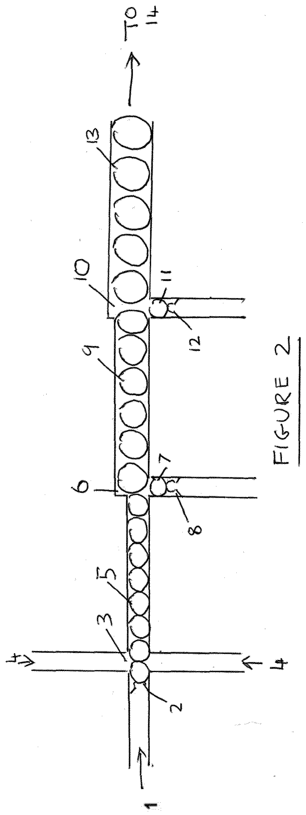

As regards other prior art, Fan et al in Nature Reviews Genetics 7(8) 632-644 (2006) provide a general review of the development of methods and platforms that have enabled highly parallel genomic assays for genotyping, copy-number measurements, sequencing and detecting loss of heterozygosity, allele-specific expression and methylation. Figure 2a of this review schematically shows the use of a circularizable probe with 3' and 5' ends that anneal upstream and downstream of a site of single nucleotide polymorphism (SNP) on an analyte thereby leaving a gap which is subsequently filled with a nucleotide which is the complement of the SNP to form a complete circular probe which may then be amplified after release. However unlike our method, the nucleotide which is captured during the filling process is not obtained directly from the analyte itself.

WO03080861 discloses a process wherein a nucleic acid analyte is subjected to progressive pyrophosphorolysis in the presence of a nucleotide-specific reactive label which attaches directly to the nucleotide as it is released. Not only is this quite different from the method we employ but in practice the fluorescence signal measured when the labelled nucleotides are subsequently interrogated would likely be too weak to enable reliable identification above the associated background noise.

Finally, WO9418218 teaches a DNA sequencing method in which the analyte is subjected to progressive exonucleolysis to generate a stream of single nucleotide diphosphates or monophosphates which are then incorporated into a fluorescence-enhancing matrix before being detected. Not only is this a completely different approach to the one we describe but we again observe that any signal generated would likely be too weak to be reliably detected and identified.

One disadvantage of the exonucleolysis-based methods described above is that the rate of generation of the fluorescence signal is limited by the speed of the digestion stage. Whilst this is by no means fatal to the utility of the method it nevertheless remains highly desirable from a technical perspective to reduce the incubation period associated with this growth in fluorescence to enable faster sequencing; especially where long sequencing reads are contemplated. This we have now achieved by replacing the exonucleolytic digestion of the used probe with a relatively faster selective endonucleolytic nicking of the strand bearing the quenched fluorophores. Thus according to the present invention there is provided a method of sequencing a nucleic acid characterised by the steps of (1) generating a stream of single nucleoside triphosphates by progressive enzymatic digestion of the nucleic acid; (2) producing at least one substantially double-stranded oligonucleotide used probe by reacting, in the presence of a polymerase and a ligase, at least one of the single nucleoside triphosphates with a corresponding biological probe comprising (a) a first single-stranded oligonucleotide including a single nucleotide capture site for capturing the single nucleoside triphosphate , a restriction endonuclease nicking site including the capture site and oligonucleotide regions juxtaposed either side of the nicking-site bearing respectively at least one fluorophore and at least one quencher so as to render the fluorophores quenched and (b) second and third single-stranded oligonucleotides capable of hybridising to complementary regions on the first oligonucleotide either side of the capture site; (3) nicking the first oligonucleotide strand of the used probe at the nicking-site with a nicking restriction endonuclease to create separate first oligonucleotide components respectively bearing the fluorophores and the quenchers; (4) separating the first oligonucleotide components generated in step (3) from the complementary strand of the used probe and (5) detecting the fluorophores on the separated oligonucleotide component bearing them.

In one preferred embodiment, steps (3) and (4) are iterated as described below to increase significantly the number of fluorophores released for detection in step (5).

Step (1) of the method of the present invention comprises generating a stream of single nucleoside triphosphates from a nucleic acid analyte by progressive enzymatic digestion. In one embodiment this can be achieved by progressive exonucleolysis of the analyte followed by the action of a kinase on the single nucleoside monophosphates obtained. Preferably however the nucleoside triphosphates are produced directly from the analyte by progressive pyrophosphorolysis. The analyte employed in this step is suitably a double-stranded polynucleotide the length of which can in principle be unlimited; for example including up to the many millions of nucleobases found in a human gene or chromosome fragment. Typically, however, the polynucleotide will be at least 50, preferably at least 150 nucleotide pairs long; suitably it will be greater than 500, greater than 1000 and in many cases thousands of nucleotide pairs long. The analyte itself is preferably RNA or DNA of natural origin (e.g. derived from a plant, animal, bacterium or a virus) although the method can also be used to sequence synthetically-produced RNA or DNA or other nucleic acids made up wholly or in part of nucleotides whose nucleobases are not commonly encountered in nature; i.e. nucleobases other than adenine (A), guanine (G), cytosine (C), thymine (T) and uracil (U). Examples of such nucleobases include 4-acetylcytidine, 5-(carboxyhydroxylmethyl)uridine, 2-O-methylcytidine, 5-carboxymethylaminomethyl-2-thiouridine, 5-carboxymethylamino-methyluridine, dihydrouridine, 2-O-methylpseudouridine, 2-O-methylguanosine, inosine, N6-isopentyladenosine, 1-methyladenosine, 1-methylpseudouridine, 1-methylguanosine, 1-methylinosine, 2,2-dimethylguanosine, 2-methyladenosine, 2-methylguanosine, 3-methylcytidine, 5-methylcytidine, N6-methyladenosine, 7-methylguanosine, 5-methylaminomethyluridine, 5-methoxyaminomethyl-2-thiouridine, 5-methoxyuridine, 5-methoxycarbonylmethyl-2-thiouridine, 5-methoxycarbonylmethyluridine, 2-methylthio-N6-isopentenyladenosine, uridine-5-oxyacetic acid-methylester, uridine-5-oxyacetic acid, wybutoxosine, wybutosine, pseudouridine, queuosine, 2-thiocytidine, 5-methyl-2-thiouridine, 2-thiouridine, 4-thiouridine, 5-methyluridine, 2-O-methyl-5-methyluridine and 2-O-methyluridine. In the case of DNA the single nucleoside triphosphates generated are deoxyribonucleoside triphosphates whilst in the case of RNA they are ribonucleoside triphosphates.

In one embodiment of the method, step (1) further comprises a first sub-step of attaching the analyte to a substrate. Typically, this substrate comprises a microfluidic surface, a micro-bead or a permeable membrane; for example one made of glass or a non-degradable polymer. Preferably, the substrate further comprises a surface specifically adapted to receive the analyte. There are many ways in which the analyte can be attached to such surfaces any of which can in principle be used in this sub-step. For example, one method involves priming a glass surface with a functionalised silane such as an epoxysilane, an aminohydrocarbylsilane or a mercaptosilane. The reactive sites so generated can then be treated with a derivative of the analyte which has been modified to include a terminal amine, succinyl or thiol group.

In another embodiment of step (1), the analyte is pyrophosphorolysed to generate a stream of single nucleoside triphosphates the order of which corresponds to that of the sequence of the analyte. Such pyrophosphorolysis may be carried out at a temperature in the range 20 to 90°C; for example in the presence of a reaction medium comprising a suitable polymerase. Preferably it is carried out under conditions of continuous flow so that the single nucleoside triphosphates are continually removed from the reaction zone as they are liberated. Most preferably, the pyrophosphorolysis is carried out by causing an aqueous buffered medium containing the enzyme and the other typical additives to continuously flow over the surface to which the analyte is bound.

In yet another embodiment, the enzyme used is one which can cause progressive 3'-5' pyrophosphorolytic degradation of the analyte to yield a stream of nucleoside triphosphates with high fidelity and at a reasonable reaction rate. Preferably this degradation rate is as fast as possible and in one embodiment is in the range 1 to 50 nucleoside triphosphates per second.

Further information about the pyrophosphorolysis reaction as applied to the digestion of polynucleotides can be found for example in J. Biol. Chem. 244 (1969) pp. 3019-3028. Suitably, the pyrophosphorolytic digestion is carried out in the presence of a medium which further comprises pyrophosphate anion and magnesium cations; preferably in millimolar concentrations.

In step (2) of the method of the present invention at least one single nucleoside triphosphate, preferably each single nucleoside triphosphate in the stream, is reacted in the presence of a polymerase and a ligase with a probe system to generate a substantially double-stranded used probe. Preferably, before this step is carried out the product of step (1) is treated with a pyrophosphatase, to hydrolyse any residual pyrophosphate to phosphate anion.

The polymerase used in this step is suitably selected from the group consisting of those which show essentially neither exo- nor endonuclease activity under the reaction conditions. Examples of polymerases which can be advantageously used include, but are not limited to, the prokaryotic pol 1 enzymes or enzyme derivatives obtained from bacteria such as *Escherichia coli* (e.g. Klenow fragment polymerase), *Thermus aquaticus* (e.g. *Taq* Pol), *Bacillus stearothermophilus, Bacillus caldovelox* and *Bacillus caldotenax.* Any suitable ligase can in principle be used in this step.

The biological probe employed in step (2) is comprised of three components; (a) a first single-stranded oligonucleotide labelled with characteristic detectable elements in an undetectable state and (b) second and third unlabelled single-stranded oligonucleotides capable of hybridising to complementary regions on the first oligonucleotide. In one embodiment the second and third oligonucleotides are discrete entities whilst in another they are linked to each other by means of a linker region. In this latter case, in one embodiment the linker region links ends of the second and third oligonucleotides. The linker region can in principle be any divalent group but is conveniently another oligonucleotide region. In one embodiment this oligonucleotide linker region is unable to hybridise substantially to the first oligonucleotide.

The first, second and third oligonucleotides are chosen so that in step (2) the second and third oligonucleotides can hybridise respectively to 3' side and 5' side flanking regions on the first oligonucleotide which themselves are juxtaposed either side of a capture site which comprises the single nucleotide whose nucleobase is complementary to the one borne by the nucleoside triphosphate to be detected. This makes the three-component probe system highly selective for that particular nucleoside triphosphate. Thus, for example, if the analyte is derived from DNA and the first, second and third oligonucleotides are comprised of deoxyribonucleotides, the capture site will be highly selective for deoxyadenosine triphosphate if the nucleotide it comprises bears a thymine nucleobase. In one useful embodiment of the invention therefore step (2) may be carried out in the presence of a probe system comprised of a plurality of probe types; for example one, two, three or four probe types each of which comprises a first oligonucleotide having a different capture site characteristic of the various different nucleobases sought and different detectable elements attached thereto.

Typically, the first oligonucleotide is up to 150 nucleotides long, preferably between 10 and 100 nucleotides. In one embodiment the second oligonucleotide is shorter than the complementary 3' side flanking region of the first oligonucleotide by at least 1 nucleotide. In another, there is a single nucleotide mismatch between the 3' end of the first oligonucleotide and the nucleotide opposite it on the second oligonucleotide to prevent the nucleoside triphosphate being captured by the polymerase at this point. Similarly, in one embodiment the third oligonucleotide is longer than the complementary 5' side flanking region of the first oligonucleotide by at least one nucleotide, while in another there is a single nucleotide mismatch between the 3' end of the third oligonucleotide and the nucleotide opposite it in the first oligonucleotide to prevent the nucleoside triphosphate being captured by the polymerase at this point.

It is a feature of the first oligonucleotide that it is provided with a region which is labelled with its own unique type of fluorophores and that these fluorophores are arranged so as to be substantially undetectable when the probe system is in an unused state. Preferably they are arranged to be essentially non-fluorescing at those wavelengths where the fluorophores are designed to be detected. Thus, although a fluorophore may exhibit general, low-level background fluorescence across a wide part of the electromagnetic spectrum, there will typically be one or a small number of specific wavelengths or wavelength envelopes where the intensity of the fluorescence is at a maximum. It is at one or more of these maxima where the fluorophore is characteristically detected that essentially no fluorescence should occur. In the context of this patent, by the term 'essentially non-fluorescing' or equivalent wording is meant that the intensity of fluorescence of the total number of fluorophores attached to the first oligonucleotide at the relevant characteristic wavelength or wavelength envelope is less than 25%; preferably less than 10%; more preferably less than 1% and most preferably less than 0.1% of the corresponding intensity of fluorescence of an equivalent number of free fluorophores.

In principle, any method can be used to ensure that in the first oligonucleotide's unused state the fluorophores are essentially non-fluorescing. In one embodiment this is achieved by disposing fluorophores and quenchers either side of the nicking-site in respectively fluorophore and quenching regions. In another it is achieved by disposing fluorophores in the two regions either side of the nicking-site and relying on the observation that when multiple fluorophores are in close proximity to each other they tend to quench each other sufficiently well that the criterion described in the previous paragraph can be achieved without the need for quenchers. In this context of this patent, what constitutes 'close proximity' between fluorophores or between fluorophores and quenchers will depend on the particular fluorophores and quenchers used and possibly the structural characteristics of the first oligonucleotide. Consequently, it is intended that this term should be construed with reference to the required outcome rather than any particular structural arrangement of the various elements within the two regions. However, and for the purposes of providing exemplification only, it is pointed out that when adjacent fluorophores or adjacent fluorophores and quenchers are separated by a distance corresponding to the characteristic Forster distance (typically less than 5nm) sufficient quenching will generally be achieved.

As regards the fluorophores themselves, they can in principle be chosen from any of those conventionally used in the art including but not limited to xanthene moieties e.g. fluorescein, rhodamine and their derivatives such as fluorescein isothiocyanate, rhodamine B and the like; coumarin moieties (e.g. hydroxy-, methyl- and aminocoumarin) and cyanine moieties such as Cy2, Cy3, Cy5 and Cy7. Specific examples include fluorophores derived from the following commonly used dyes: Alexa dyes, cyanine dyes, Atto Tec dyes, and rhodamine dyes. Examples also include: Atto 633 (ATTO-TEC GmbH), Texas Red™, Atto 740 (ATTO-TEC GmbH), Rose Bengal, Alexa Fluor™ 750 C₅-maleimide (Invitrogen), Alexa Fluor™ 532 C₂-maleimide (Invitrogen) and Rhodamine Red C₂-maleimide and Rhodamine Green as well as phosphoramadite dyes such as Quasar 570. Alternatively, a quantum dot or a near infra-red dye such as those supplied by LI-COR Biosciences can be employed. The fluorophore is typically attached to the first oligonucleotide via a nucleotide base using chemical methods known in the art.

Suitable quenchers include those which work by a Forster resonance energy transfer (FRET) mechanism. Examples of commercially available quenchers which can be used in association with the above mentioned-fluorophores include but are not limited to DDQ-1, Dabcyl, Eclipse, Iowa Black FQ and RQ, IR Dye-QC1, BHQ-0, BHQ-1, -2 and -3 and QSY-7 and -21.

It is a feature of the first oligonucleotide that the fluorophores are rendered undetectable in the probe's unused state by virtue of fluorophore and quencher regions juxtaposed either side of a single-stranded restriction endonuclease nicking-site adapted to undergo selective, nicking endonucleolysis when the probe has been used and is in a double-stranded state. In one preferred embodiment, this nicking-site comprises an oligonucleotide region on the first oligonucleotide which itself includes the capture site. In another embodiment, where more than one probe system is employed, it is preferred that each differently labelled first oligonucleotide nevertheless comprise a common nicking-site so that only one nicking restriction endonuclease need be employed. To achieve this, it is therefore preferred that the nicking-site will be comprised of a sequence containing at least one of each of the typical nucleotides of RNA or DNA as the case may be.

In another preferred embodiment, the nicking restriction endonuclease as defined herein is a conventional restriction endonuclease otherwise capable of cleaving both strands of the used probe and in the region of the nicking-site the complementary strand is rendered resistant to endonucleolysis; for example, by including one or more endonucleolytic blocking sites. In one embodiment, these blocking groups may be selected from phosphorothioate linkages and other backbone modifications commonly used in the art, C3 spacers, phosphate groups, or the like.

Step (2) is suitably carried out by contacting each single nucleoside triphosphate in the stream with the enzymes and one or more probes as described above at a temperature in the range 20 to 80°C.

The product of step (2) of the method of the invention is, as mentioned above, a substantially double-stranded used probe whose constituent strands are respectively the first oligonucleotide and a complementary oligonucleotide comprised of the second oligonucleotide, a nucleotide derived from the single nucleoside triphosphate and finally the third oligonucleotide. If the second and third oligonucleotides have previously been joined together by a linker region then it will be readily apparent that this complimentary oligonucleotide will comprise a closed-loop strand.

In step (3) the used probe is treated with a nicking restriction endonuclease at a temperature in the range 30 to 100°C. In this step the strand of the used probe derived from the first oligonucleotide is severed into two separate components which can then in step (4) be de-hybridised from the probe's complementary strand thereby separating the fluorophore and quencher regions from each other and allowing the fluorophores to fluoresce. Thus, as endonucleolysis and de-hybridisation occurs, the observer sees a rapid growth in the fluorescence signal. The characteristics of this fluorescence then indirectly reflects the nature of the single nucleoside triphosphate originally captured by the relevant probe system. Step (4) can most effectively be achieved by heating the nicked used probe to a temperature in the range 30 to 100°C.

Thereafter, and in step (5), the fluorophores liberated in step (4) or via the various iterations of step (3) and (4) are detected and the nature of the nucleobase attached to the single nucleoside triphosphate determined by inference. By carrying out the method of the invention systematically for all the single nucleoside triphosphates in the stream generated in step (1), data characteristic of the sequence of original nucleic acid analyte can be generated and analysed. Methods of doing this are well-known in the art; for example the reaction medium can be interrogated with light from a laser and any fluorescence generated detected using a photodetector or an equivalent device tuned to the characteristic fluorescence wavelength(s) or wavelength envelope(s) of the various fluorophores. This in turn causes the photodetector to generate a characteristic electrical signal which can be processed and analysed in a computer using known algorithms.

As mentioned above, in one preferred embodiment, at the end of step (4) the complementary oligonucleotide derived from the used probe, now present in single-stranded form, is caused to hybridise to another corresponding first oligonucleotide molecule thereby producing a new substantially double-stranded oligonucleotide product corresponding to, i.e. having the same chemical and physical structure, as the used probe. This product is then nicked in a repeat of step (3) thereby releasing further fluorophores in a detectable state and again regenerating the complementary oligonucleotide after a repeat of step (4). Thereafter, steps (3) and (4) are allowed to iterate causing further enhancement in the fluorescence signal; in principle until substantially all of the first oligonucleotide has been consumed. As a consequence the observer sees a much greater enhancement of the fluorescence signal than might otherwise have been obtained.

In one particularly preferred embodiment, the method of the present invention is carried out wholly or partially in a stream of microdroplets, at least some of which contain a single nucleoside triphosphate; suitably an ordered stream. Such a method may begin, for example, by inserting the nucleoside triphosphates generated in step (1) one-by-one into a corresponding stream of aqueous microdroplets maintained in an immiscible carrier solvent such as a hydrocarbon or silicone oil to help preserve the ordering. Alternatively, this can be achieved by directly creating the microdroplets downstream of the digestion (pyrophosphorolysis) zone; for example, by causing the reaction medium to emerge from a microdroplet head of suitable dimensions into a flowing stream of the solvent. Alternatively, small aliquots of the reaction medium from step (1) can be regularly and sequentially injected into a stream of pre-existing aqueous microdroplets suspended in the solvent. If this latter approach is adopted, each microdroplet may already contain the various components of the probe system(s) together with the enzymes and any other reagents (e.g. buffer) required to effect steps (2) to (4). In yet another approach, the microdroplets created in the former embodiment can be caused to coalesce subsequently with a stream of such pre-existing microdroplets to achieve a similar outcome. In these microdroplet methods, step (5) then preferably involves delivering the microdroplets to a storage area and interrogating each microdroplet to identify the fluorophores liberated. Thereafter the results obtained from each microdroplet are assembled into a stream of data characteristic of the sequence of the original nucleic acid analyte.

To avoid the risk that a given microdroplet contains more than one nucleoside triphosphate it is preferred to release each nucleoside triphosphate in step (1) at a rate such that each filled microdroplet is separated on average by from 1 to 20 preferably 2 to 10 empty ones. Thereafter the stream of filled and unfilled microdroplets in the solvent is caused to flow along a flow path, suitably a microfluidic flow path, at a rate and in a manner such that they are maintained in a discrete state and do not have the opportunity to coalesce with each other. Suitably the microdroplets employed have a finite diameter less than 100 microns, preferably less than 50 microns, more preferably less than 20 microns and even more preferably less than 15 microns. Most preferably of all their diameters are in the range 2 to 20 microns. In one embodiment, the microdroplet flow rate through the whole system is in the range 50 to 3000 microdroplets per second preferably 100 to 2000.

In a second aspect of the invention there is also provided a multi-component biological probe characterised by comprising (a) a first single-stranded oligonucleotide including a single nucleotide capture site for capturing a single nucleoside triphosphate, a restriction endonuclease nicking site including the capture site and oligonucleotide regions juxtaposed either side of the nicking-site bearing respectively at least one fluorophore and at least one quencher so as to render the fluorophores quenched and (b) second and third single-stranded oligonucleotides capable of hybridising to complementary regions on the first oligonucleotide either side of the capture site.

In one embodiment the oligonucleotide regions comprise a fluorophore region where at least one fluorophore is borne and a quencher region where at least one quencher is borne. In another embodiment the two regions both bear fluorophores or a mixture of fluorophores and quenchers. In this case all the fluorophores are arranged to self-quench one another. Suitable fluorophores and quenchers include but are not limited to those described above. In another embodiment the second and third oligonucleotides are connected by a linker region as explained above; with the linker region itself preferably being another oligonucleotide region. Preferably, the nicking endonuclease recognition site on the first oligonucleotide includes the capture site.

As explained above, for the purposes of DNA or RNA sequencing, the biological probes described herein can be assembled into a corresponding biological probe system comprised of from one to four different first oligonucleotide types differing only in the nucleobase characteristic of the capture site (A, G, C, and T or U) and the fluorophores to be used. In another embodiment the biological probe system is made manifest as a kit further comprising at least one of a ligase, a polymerase and a nicking restriction endonuclease capable of nicking the nicking-site once a double-stranded used probe within the probe system has been created.

Details of suitable nicking restriction endonucleases which can be used with the method, probes and probe systems of the present invention can be found at http://rebase.neb.com in the database associated therewith.

It is believed that the detection method used in the sequencing method described above is also generally applicable to the analysis, characterisation or quantification of single nucleoside triphosphates in a biological sample or an analyte derived therefrom. Thus, in a third aspect of the present invention there is provided a method of analysing a single nucleoside triphosphate characterised by the steps of (1) producing at least one substantially double-stranded oligonucleotide used probe by reacting, in the presence of a polymerase and a ligase, the single nucleoside triphosphate with a corresponding biological probe comprising (a) a first single-stranded oligonucleotide including a single nucleotide capture site for capturing the single nucleoside triphosphate, a restriction endonuclease nicking site including the capture site and oligonucleotide regions juxtaposed either side of the nicking-site bearing respectively at least one fluorophore and at least one quencher so as to render the fluorophores quenched and (b) second and third single-stranded oligonucleotides capable of hybridising to complementary regions on the first oligonucleotide either side of the capture site; (2) nicking the first oligonucleotide strand of the used probe at the nicking-site with a nicking restriction endonuclease to create separate first oligonucleotide components respectively bearing the fluorophores and the quenchers; (3) separating the first oligonucleotide components generated in step (2) from the complementary strand of the used probe and (4) detecting the fluorophores on the separated oligonucleotide component bearing them.

In such a method the nature of the various steps and the biological probes employed will suitably be as described above. In one embodiment the single nucleoside triphosphate will be derived from a precursor double-stranded DNA analyte by pyrophosphorolysis. In another it will be generated from a precursor single nucleoside monophosphate or single nucleoside diphosphate using for example a kinase (see for example Biotechnology and Bioengineering by Bao and Ryu (DOI 10.1002/bit.21498)).

The invention is now illustrated with reference to the following Examples.

### Example 1 - Preparation and use of a probe system

A single-stranded first oligonucleotide 1 was prepared, having the following nucleotide sequence:
5'-ATGTTCTGATATCXCGTATTCATTGCQTGATGCCCTTAGC-3'
wherein A, C, G, and T represent nucleotides bearing the relevant characteristic nucleotide base of DNA; X represents a deoxythymidine nucleotide (T) labelled with Atto 655 dye using conventional amine attachment chemistry and Q represents a deoxythymidine nucleotide labelled with a BHQ-2 quencher. It further comprises a capture region (C nucleotide) at the 21^{st} base from its 5' end, selective for capturing deoxyguanosine triphosphate nucleotides (dGTPs) in a mixture of deoxynucleoside triphosphates (dNTPs), and the recognition sequence for the nicking endonuclease Nb.BsrDI, 'NNCATTGC'.

Another single-stranded oligonucleotide 2, comprising an oligonucleotide region having a sequence complementary to the 3' end of the first oligonucleotide with a four base mismatch, and a single-stranded oligonucleotide 3, comprising an oligonucleotide region having a sequence complementary to the 5' end of the first oligonucleotide with a four base mismatch and a 5' phosphate group, were also prepared. They had the following nucleotide sequences:
Oligonucleotide 2: 5'-TTCCGCATCAAGCAAT-3'
Oligonucleotide 3: 5'-PAATACGAGATATGTCT-3'
wherein P represents the 5' phosphate group.

A reaction mixture comprising the probe system was then prepared. It had a composition corresponding to that derived from the following formulation:
20uL 5x buffer pH 7.9
10uL oligonucleotide 1, 1000 nM
10uL oligonucleotide 2, 10 nM
10uL oligonucleotide 3, 10nM
10uL spermine solution, 10mM
10U Nb.BsrDI nicking endonuclease (ex. New England Biolabs Inc.)
20U E. Coli ligase
5.8U Bst Large Fragment polymerase
10uL mixture of dNTPs, 1 nM
Water to 100uL
wherein the 5x buffer comprised the following mixture:
25uL Trizma Acetate, 1M, pH 7.9
50uL aqueous Magnesium Acetate, 1M
25uL aqueous Potassium Acetate, 1M
50uL Triton X-100 surfactant (10%)
500µg BSA
Water to 1mL

Capture of the dGTPs and ligation of oligonucleotide 2 to oligonucleotide 3 to form a used probe was then carried out by incubating the mixture at 37°C for 30 minutes after which the temperature was increased to 60°C for a further 120 minutes. The reaction mixture was illuminated using the 633nm line of a Helium-Neon laser and the resulting characteristic fluorescence of the Atto 655 dye detected using a camera as the cycle of endonucleolysis and regeneration of the used probe occurred.

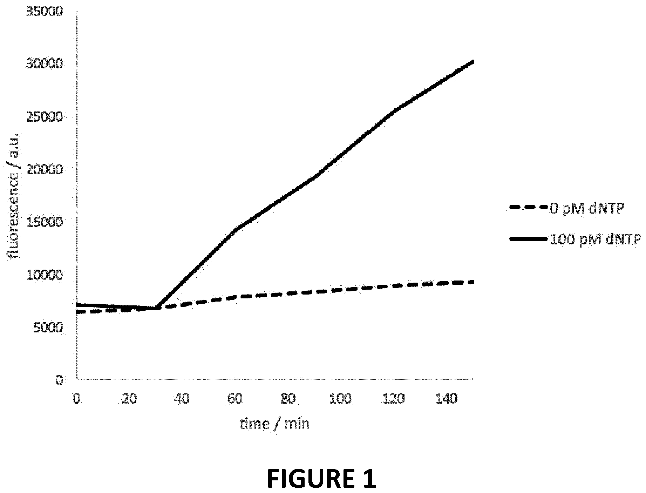

The growth in intensity of fluorescence over time in the presence and absence of the dNTP component of the reaction was monitored and the results shown graphically in figure 1. From this it can be seen that the probe system efficiently captures the dGTPs and the cyclic nature of the process of the present invention leads to a rapid growth in fluorescence signal. On the contrary, in a comparative experiment where no dNTPs were present in the reaction mixture the Atto 655 dye on oligonucleotide 1 did not exhibit fluorescence to any significant extent.

### Example 2 - Droplet microfluidic method using the probe system

Figure 2 schematically illustrates a microfluidic sequencing device in which microdroplets each containing a single nucleotide base are made to undergo reaction with a probe system of the type above as described above.

An aqueous medium 1 comprising a stream of single nucleotide triphosphates obtained by the progressive pyrophosphorolysis of a 100 nucleotide base polynucleotide analyte derived from human DNA is caused to flow through a ten micron diameter microfluidic tube fabricated from PDMS polymer. The pyrophosphorolysis reaction itself is carried out by passing a stream of an aqueous, buffered (pH 7.5) reaction medium at 72°C, comprising *Taq Pol* and a solution having a 2 millimoles per litre concentration of each of sodium pyrophosphate and magnesium chloride, over a glass micro bead onto which the analyte has been previously attached by means of a succinyl bridge. The order of the single nucleotides in 1, which is downstream of the micro bead, corresponds to the sequence of the analyte. 1 emerges from a droplet head 2 into a first chamber 3 where it is contacted with one or more streams of immiscible light silicone oil 4. The velocities of these streams are chosen to avoid turbulent mixing and to create aqueous spherical droplets 5 suspended in the oil each having a diameter of approximately eight microns. Typically, rates are adjusted so that between adjacent filled droplets there are on average 10 empty ones. A stream of 5 is then carried forward along a second microfluidic tube of the same diameter to a second chamber 6 into which a second stream of five micron aqueous spherical droplets 7 is also fed by means of a second droplet head 8. Droplets 5 and 7 are caused to coalesce in a sequential fashion to form enlarged aqueous droplets 9 approximately nine microns in diameter. Each of 7 contains inorganic pyrophosphatase to destroy any residual pyrophosphate anion present in each of 5.

A stream of 9 is then carried forward at the same rate via microfluidic tubing into a third chamber 10 where these droplets are contacted with a third stream of five micron aqueous spherical droplets 11 also fed thereto through a corresponding droplet head 12. The time taken for each of 9 to move between chambers 6 and 10 is c.2minutes.

Droplets 9 and 11 are then caused to coalesce in 10 to produce droplets 13 (approximately ten microns in diameter). Each of 11 contains a mesophilic ligase, a thermophilic polymerase, the nicking restriction endonuclease Nb.BsrDI (ex. New England Biolabs Inc.) and a probe system comprising four sets of single-stranded oligonucleotides similar to those described in Example 1 each having a different first oligonucleotide labelled with a different fluorophore.

The stream of the coalesced microdroplets 13 so formed is then subjected to incubation at 37°C for 30 minutes followed by 60°C for 120 minutes. At the end of this time 13 is transferred to the detection system, 14.

The detection system (not shown) typically comprises a detection window in which each droplet is interrogated with incident light from a laser. Action of this light then causes the released fluorophores in each droplet to fluoresce in a way characteristic of the single nucleotide base which was originally incorporated into the captured molecule (or essentially not at all if the droplet was originally empty). The presence or absence of this fluorescence is then detected at the four characteristic wavelengths of the four fluorophores associated with the four oligonucleotide sets mentioned above. Thus as the droplets are interrogated in turn the sequence of nucleotide bases in the original polynucleotide analyte can in effect be read off.

## Claims

1. A method of sequencing a nucleic acid **characterised by** the steps of (1) generating a stream of single nucleoside triphosphates by progressive enzymatic digestion of the nucleic acid; (2) producing at least one substantially double-stranded oligonucleotide used probe by reacting, in the presence of a polymerase and a ligase, at least one of the single nucleoside triphosphates with a corresponding biological probe comprising (a) a first single-stranded oligonucleotide including a single nucleotide capture site for capturing the single nucleoside triphosphate, a restriction endonuclease nicking site including the capture site and oligonucleotide regions juxtaposed either side of the nicking-site bearing respectively at least one fluorophore and at least one quencher so as to render the fluorophores quenched and (b) second and third single-stranded oligonucleotides capable of hybridising to complementary regions on the first oligonucleotide either side of the capture site; (3) nicking the first oligonucleotide strand of the used probe at the nicking-site with a nicking restriction endonuclease to create separate first oligonucleotide components respectively bearing the fluorophores and the quenchers; (4) separating the first oligonucleotide components generated in step (3) from the complementary strand of the used probe and (5) detecting the fluorophores on the separated oligonucleotide component bearing them.

2. A method as claimed in claim 1 **characterised by** further comprising between steps (4) and (5) the step of reacting the separated complementary strand of the used probe with another first oligonucleotide to produce a further substantially double-stranded oligonucleotide product identical to the used probe and iterating steps (3) and (4).

3. A method as claimed in claim 1 or claim 2 **characterised in that** the quencher region comprises fluorophores identical to those borne by the fluorophore region and that all the fluorophores are arranged to quench one another.

4. A method as claimed in any of the preceding claims **characterised in that** the second oligonucleotide and the third oligonucleotide are connected by a linker region.

5. A method as claimed in claim 4 **characterised in that** the linker region comprises an oligonucleotide region.

6. A method as claimed in claim 4 or 5 **characterised in that** the complementary strand of the used probe comprises a closed-loop.

7. A method as claimed in any of the preceding claims **characterised in that** an endonuclease adapted to cleave both strands of the used probe is employed and the complementary strand of the used probe is rendered resistant to endonucleolysis.

8. A method as claimed in any of the preceding claims **characterised in that** the probe system comprises a plurality of first oligonucleotide types each provided with a different capture site and characteristic detectable elements.

9. A method as claimed in claim 8 **characterised in that** up to four different sets of oligonucleotide probe systems are employed, the first oligonucleotide of each set having a capture site selective for one of the characteristic nucleotide bases of naturally-occurring DNA or RNA and different detectable elements.

10. A method as claimed in any of the preceding claims **characterised in that** step (1) further comprises containing each single nucleoside triphosphate in a corresponding microdroplet and that steps (2) to (5) are carried out in each microdroplet.

11. A multi-component biological probe **characterised by** comprising (a) a first single-stranded oligonucleotide including a single nucleotide capture site for capturing a single nucleoside triphosphate; a restriction endonuclease nicking site including the capture site and oligonucleotide regions juxtaposed either side of the nicking-site bearing respectively at least one fluorophore and at least one quencher so as to render the fluorophores quenched and (b) second and third single-stranded oligonucleotides capable of hybridising to complementary regions on the first oligonucleotide either side of the capture site.

12. A biological probe as claimed in claim 11 **characterised in that** the quencher region comprises fluorophores identical to those borne by the fluorophore region and that all the fluorophores are arranged to self-quench one another.

13. A biological probe as claimed in claim 11 or claim 12 **characterised in that** the second oligonucleotide and third oligonucleotides are connected by an oligonucleotide linker region.

14. A biological probe as claimed in any of claims 11 to 13 **characterised in that** the nicking endonuclease recognition site on the first oligonucleotide includes the capture site.

15. A method of analysing a single nucleoside triphosphate **characterised by** the steps of (1) producing at least one substantially double-stranded oligonucleotide used probe by reacting, in the presence of a polymerase and a ligase, the single nucleoside triphosphate with a corresponding biological probe comprising (a) a first single-stranded oligonucleotide including a single nucleotide capture site for capturing the single nucleoside triphosphate, a restriction endonuclease nicking site including the capture site and oligonucleotide regions juxtaposed either side of the nicking-site bearing respectively at least one fluorophore and at least one quencher so as to render the fluorophores quenched and (b) second and third single-stranded oligonucleotides capable of hybridising to complementary regions on the first oligonucleotide either side of the capture site; (2) nicking the first oligonucleotide strand of the used probe at the nicking-site with a nicking restriction endonuclease to create separate first oligonucleotide components respectively bearing the fluorophores and the quenchers; (3) separating the first oligonucleotide components generated in step (2) from the complementary strand of the used probe and (4) detecting the fluorophores on the separated oligonucleotide component bearing them.

## Patentansprüche

1. Verfahren zur Sequenzierung einer Nukleinsäure, **gekennzeichnet durch** die Schritte (1) Erzeugen eines Stroms von Einzelnukleosidtriphosphaten durch progressive enzymatische Verdauung der Nukleinsäure; (2) Herstellen wenigstens einer weitgehend doppelsträngigen benutzten Oligonukleotidsonde durch Umsetzen, in Gegenwart einer Polymerase und einer Ligase, wenigstens eines der Einzelnukleosidtriphosphate mit einer entsprechenden biologischen Sonde, die (a) ein erstes einzelsträngiges Oligonukleotid, das eine Einzelnukleotideinfangstelle zum Einfangen des Einzelnukleosidtriphosphats, eine Restriktionsendonuklease-Nicking-Stelle, die die Einfangstelle enthält, und auf jeder Seite neben der Nicking-Stelle liegende Oligonukleotidregionen, die wenigstens einen Fluorophor bzw. wenigstens einen Quencher tragen, so dass die Fluorophore im Quenched-Zustand vorliegen, enthält, und (b) zweite und dritte einzelsträngige Oligonukleotide mit der Fähigkeit zur Hybridisierung an komplementäre Regionen auf dem ersten Oligonukleotid zu beiden Seiten der Einfangstelle umfasst; (3) Nicking des ersten Oligonukleotidstrangs der benutzten Sonde an der Nicking-Stelle mit einer Nicking-Restriktionsendonuklease, so dass getrennte Erstes-Oligonukleotid-Bestandteile erzeugt werden, die die Fluorophore bzw. die Quencher tragen; (4) Trennen der in Schritt (3) erzeugten Erstes-Oligonukleotid-Bestandteile vom Komplementärstrang der benutzten Sonde und (5) Nachweisen der Fluorophore auf dem sie tragenden getrennten Oligonukleotid-Bestandteil.

2. Verfahren nach Anspruch 1, **gekennzeichnet durch** ferner umfassend zwischen Schritt (4) und (5) den Schritt, bei dem der getrennte Komplementärstrang der benutzten Sonde mit einem weiteren ersten Oligonukleotid umgesetzt wird, so dass ein weiteres weitgehend doppelsträngiges Oligonukleotidprodukt entsteht, das mit der benutzten Sonde identisch ist, und Schritte (3) und (4) wiederholt werden.

3. Verfahren nach Anspruch 1 oder Anspruch 2, **dadurch gekennzeichnet, dass** die Quencherregion Fluorophore umfasst, die mit den von der Fluorophorregion getragenen identisch sind, und dass alle Fluorophore so angeordnet sind, dass gegenseitiges Quenching stattfindet.

4. Verfahren nach einem der vorhergehenden Ansprüche, **dadurch gekennzeichnet, dass** das zweite Oligonukleotid und das dritte Oligonukleotid über eine Linkerregion verbunden sind.

5. Verfahren nach Anspruch 4, **dadurch gekennzeichnet, dass** die Linkerregion eine Oligonukleotidregion umfasst.

6. Verfahren nach Anspruch 4 oder 5, **dadurch gekennzeichnet, dass** der Komplementärstrang der benutzten Sonde eine Closed-loop umfasst.

7. Verfahren nach einem der vorhergehenden Ansprüche, **dadurch gekennzeichnet, dass** eine zur Spaltung beider Stränge der benutzten Sonde adaptierte Endonuklease eingesetzt und der Komplementärstrang der benutzten Sonde resistent gegen Endonukleolyse gemacht wird.

8. Verfahren nach einem der vorhergehenden Ansprüche, **dadurch gekennzeichnet, dass** das Sondensystem mehrere Erstes-Oligonukleotid-Typen umfasst, die jeweils mit einer unterschiedlichen Einfangstelle und charakteristischen nachweisbaren Elementen versehen sind.

9. Verfahren nach Anspruch 8, **dadurch gekennzeichnet, dass** bis zu vier unterschiedliche Sätze von Oligonukleotidsondensystemen eingesetzt werden, wobei das erste Oligonukleotid von jedem Satz eine Einfangstelle, die für eine der charakteristischen Nukleotidbasen natürlich vorkommender DNA oder RNA selektiv ist, und unterschiedliche nachweisbare Elemente aufweist.

10. Verfahren nach einem der vorhergehenden Ansprüche, **dadurch gekennzeichnet, dass** Schritt (1) ferner umfasst, dass die Einzelnukleosidtriphosphate jeweils in einem entsprechenden Mikrotröpfchen enthalten sind, und dass Schritte (2) bis (5) in jedem Mikrotröpfchen durchgeführt werden.

11. Biologische Mehrkomponentensonde, **gekennzeichnet durch** umfassend (a) ein erstes einzelsträngiges Oligonukleotid, das eine Einzelnukleotideinfangstelle zum Einfangen des Einzelnukleosidtriphosphats; eine Restriktionsendonuklease-Nicking-Stelle, die die Einfangstelle enthält, und auf jeder Seite neben der Nicking-Stelle liegende Oligonukleotidregionen, die wenigstens einen Fluorophor bzw. wenigstens einen Quencher tragen, so dass die Fluorophore im Quenched-Zustand vorliegen, enthält, und (b) zweite und dritte einzelsträngige Oligonukleotide mit der Fähigkeit zur Hybridisierung an komplementäre Regionen auf dem ersten Oligonukleotid zu beiden Seiten der Einfangstelle.

12. Biologische Sonde nach Anspruch 11, **dadurch gekennzeichnet, dass** die Quencherregion Fluorophore umfasst, die mit den von der Fluorophorregion getragenen identisch sind, und dass alle Fluorophore so angeordnet sind, dass gegenseitiges Selbst-Quenching stattfindet.

13. Biologische Sonde nach Anspruch 11 oder Anspruch 12, **dadurch gekennzeichnet, dass** das zweite Oligonukleotid und dritte Oligonukleotide über eine Oligonukleotidlinkerregion verbunden sind.

14. Biologische Sonde nach einem der Ansprüche 11 bis 13, **dadurch gekennzeichnet, dass** die Nicking-Endonuklease-Erkennungsstelle auf dem ersten Oligonukleotid die Einfangstelle enthält.

15. Verfahren zur Analyse eines Einzelnukleosidtriphosphats, **gekennzeichnet durch** die Schritte (1) Herstellen wenigstens einer weitgehend doppelsträngigen benutzten Oligonukleotidsonde durch Umsetzen, in Gegenwart einer Polymerase und einer Ligase, des Einzelnukleosidtriphosphats mit einer entsprechenden biologischen Sonde, die (a) ein erstes einzelsträngiges Oligonukleotid, das eine Einzelnukleotideinfangstelle zum Einfangen des Einzelnukleosidtriphosphats, eine Restriktionsendonuklease-Nicking-Stelle, die die Einfangstelle enthält, und auf jeder Seite neben der Nicking-Stelle liegende Oligonukleotidregionen, die wenigstens einen Fluorophor bzw. wenigstens einen Quencher tragen, so dass die Fluorophore im Quenched-Zustand vorliegen, enthält, und (b) zweite und dritte einzelsträngige Oligonukleotide mit der Fähigkeit zur Hybridisierung an komplementäre Regionen auf dem ersten Oligonukleotid zu beiden Seiten der Einfangstelle umfasst; (2) Nicking des ersten Oligonukleotidstrangs der benutzten Sonde an der Nicking-Stelle mit einer Nicking-Restriktionsendonuklease, so dass getrennte Erstes-Oligonukleotid-Bestandteile erzeugt werden, die die Fluorophore bzw. die Quencher tragen; (3) Trennen der in Schritt (2) erzeugten Erstes-Oligonukleotid-Bestandteile vom Komplementärstrang der benutzten Sonde und (4) Nachweisen der Fluorophore auf dem sie tragenden getrennten Oligonukleotid-Bestandteil.

## Revendications

1. Procédé de séquençage d'un acide nucléique **caractérisé par** les étapes de (1) génération d'un flux de nucléosides triphosphates uniques par digestion enzymatique progressive de l'acide nucléique ; (2) production d'au moins une sonde oligonucléotidique sensiblement double brin utilisée par réaction, en présence d'une polymérase et d'une ligase, d'au moins un des nucléosides triphosphates uniques avec une sonde biologique correspondante comprenant (a) un premier oligonucléotide simple brin comprenant un site de capture de nucléotide unique pour capturer le nucléoside triphosphate unique, un site de coupure simple brin d'endonucléase de restriction comprenant les régions de site de capture et oligonucléotidique juxtaposées de chaque côté du site de coupure simple brin portant respectivement au moins un fluorophore et au moins un désactiveur de façon à rendre les fluorophores désactivés et (b) des deuxième et troisième oligonucléotides simples brins capables de s'hybrider à des régions complémentaire sur le premier oligonucléotide de chaque côté du site de capture ; (3) coupure simple brin du premier brin oligonucléotidique de la sonde utilisée au site de coupure simple brin avec une endonucléase de restriction de coupure simple brin pour créer des premiers composants oligonucléotidiques séparés portant respectivement les fluorophores et les désactiveurs ; (4) séparation des premiers composants oligonucléotidiques générés dans l'étape (3) du brin complémentaire de la sonde utilisée et (5) détection des fluorophores sur le composant oligonucléotidique séparé portant ceux-ci.

2. Procédé selon la revendication 1, **caractérisé en ce qu'**il comprend en outre, entre les étapes (4) et (5), l'étape de réaction du brin complémentaire séparé de la sonde utilisée avec un autre premier oligonucléotide pour produire un autre produit oligonucléotidique sensiblement double brin identique à la sonde utilisée et l'itération des étapes (3) et (4).

3. Procédé selon la revendication 1 ou la revendication 2, **caractérisé en ce que** la région de désactiveur comprend des fluorophores identiques à ceux portés par la région de fluorophore et que tous les fluorophores sont agencés de façon à se désactiver mutuellement.

4. Procédé selon l'une quelconque des revendications précédentes, **caractérisé en ce que** le deuxième oligonucléotide et le troisième oligonucléotide sont reliés par une région de lieur.

5. Procédé selon la revendication 4, **caractérisé en ce que** la région de lieur comprend une région d'oligonucléotide.

6. Procédé selon la revendication 4 ou 5, **caractérisé en ce que** le brin complémentaire de la sonde utilisée comprend une boucle fermée.

7. Procédé selon l'une quelconque des revendications précédentes, **caractérisé en ce qu'**une endonucléase adaptée pour cliver les deux brins de la sonde utilisée est utilisée et le brin complémentaire de la sonde utilisée est rendu résistant à l'endonucléolyse.

8. Procédé selon l'une quelconque des revendications précédentes, **caractérisé en ce que** le système de sonde comprend une pluralité de types de premier oligonucléotide, chacun pourvu d'un site de capture différent et d'éléments détectables caractéristiques.

9. Procédé selon la revendication 8, **caractérisé en ce que** jusqu'à quatre ensembles différents de systèmes de sonde oligonucléotidique sont utilisés, le premier oligonucléotide de chaque ensemble ayant un site de capture sélectif pour l'une des bases nucléotidiques caractéristiques d'ADN ou d'ARN d'origine naturelle et différents éléments détectables.

10. Procédé selon l'une quelconque des revendications précédentes, **caractérisé en ce que** l'étape (1) comprend en outre l'inclusion de chaque nucléoside triphosphate unique dans une microgouttelette correspondante et que les étapes (2) à (5) sont conduites dans chaque microgouttelette.

11. Sonde biologique multicomposant **caractérisée en ce qu'**elle comprend (a) un premier oligonucléotide simple brin comprenant un site de capture de nucléotide unique pour capturer un nucléoside triphosphate unique ; un site de coupure simple brin d'endonucléase de restriction comprenant le site de capture et des régions d'oligonucléotide juxtaposées de chaque côté du site de coupure simple brin portant respectivement au moins un fluorophore et au moins un désactiveur de façon à rendre les fluorophores désactivés et (b) des deuxième et troisième oligonucléotides simples brins capables de s'hybrider à des régions complémentaires sur le premier oligonucléotide de chaque côté du site de capture.

12. Sonde biologique selon la revendication 11, **caractérisée en ce que** la région de désactiveur comprend des fluorophores identiques à ceux portés par la région de fluorophore et que tous les fluorophores sont agencés pour s'autodésactiver mutuellement.

13. Sonde biologique selon la revendication 11 ou la revendication 12, **caractérisée en ce que** le deuxième oligonucléotide et le troisième oligonucléotide sont reliés par une région de lieur oligonucléotidique.

14. Sonde biologique selon l'une quelconque des revendications 11 à 13, **caractérisée en ce que** le site de reconnaissance d'endonucléase de coupure simple brin sur le premier oligonucléotide comprend le site de capture.

15. Procédé d'analyse d'un nucléoside triphosphate unique **caractérisé par** les étapes de (1) production d'au moins une sonde oligonucléotidique sensiblement double brin utilisée par réaction, en présence d'une polymérase et d'une ligase, du nucléoside triphosphate unique avec une sonde biologique correspondante comprenant (a) un premier oligonucléotide simple brin comprenant un site de capture de nucléotide unique pour capturer le nucléoside triphosphate unique, un site de coupure simple brin d'endonucléase de restriction comprenant les régions de site de capture et oligonucléotidique juxtaposées de chaque côté du site de coupure simple brin portant respectivement au moins un fluorophore et au moins un désactiveur de façon à rendre les fluorophores désactivés et (b) des deuxième et troisième oligonucléotides simples brins capables de s'hybrider à des régions complémentaires sur le premier oligonucléotide de chaque côté du site de capture ; (2) coupure simple brin du premier brin oligonucléotidique de la sonde utilisée au site de coupure simple brin avec une endonucléase de restriction de coupure simple brin pour créer des premiers composants oligonucléotidiques séparés portant respectivement les fluorophores et les désactiveurs ; (3) séparation des premiers composants oligonucléotidiques générés dans l'étape (2) du brin complémentaire de la sonde utilisée et (4) détection des fluorophores sur le composant oligonucléotidique séparé portant ceux-ci.
